Europäisches Patentamt

European Patent Office

Office européen des brevets

(11) Publication number: **0 146 306**
**B1**

# (12) EUROPEAN PATENT SPECIFICATION

(45) Date of publication of patent specification: **15.03.89**

(21) Application number: **84308411.2**

(22) Date of filing: **04.12.84**

(51) Int. Cl.⁴: **C 07 D 487/04** // A61K31/55

(54) Adinazolam methanesulfonate.

(30) Priority: **19.12.83 US 562685**

(43) Date of publication of application:
**26.06.85 Bulletin 85/26**

(45) Publication of the grant of the patent:
**15.03.89 Bulletin 89/11**

(84) Designated Contracting States:
**BE CH DE FR GB IT LI NL SE**

(56) References cited:
**DE-A-2 201 210**
**DE-A-2 548 737**
**DE-A-2 709 842**
**US-A-4 250 094**

(73) Proprietor: **THE UPJOHN COMPANY**
**301 Henrietta Street**
**Kalamazoo, Michigan 49001 (US)**

(72) Inventor: **Nichols, Steve**
**c/o The Upjohn Company 301 Henrietta Street**
**Kalamazoo, MI 49001 (US)**
Inventor: **Meulman, Paul Allen**
**c/o The Upjohn Company 301 Henrietta Street**
**Kalamazoo, MI 49001 (US)**
Inventor: **Tiffany, Burris Dwight**
**c/o The Upjohn Company, 301 Henrietta Street**
**Kalamazoo, MI 49001 (US)**

(74) Representative: **Perry, Robert Edward et al**
**GILL JENNINGS & EVERY 53-64 Chancery Lane**
**London WC2A 1HN (GB)**

The file contains technical information
submitted after the application was filed and
not included in this specification

Courier Press, Leamington Spa, England.

# EP 0 146 306 B1

## Description

This invention relates to a salt form of adinazolam, and its preparation. Adinazolam is 8-chloro-1-(dimethylamino)methyl-6-phenyl-4H-s-triazolo[4,3-a][1,4]benzodiazepine.

Adinazolam and its methane sulfonate salt are described and claimed in US—A—4250094. The salt is being clinically studied for use in the treatment of human patients suffering from acute depression and psychotic disorders including schizophrenia.

To be in a commercially-acceptable form, adinazolam methanesulfonate should be in a stable, crystalline form which can stand in bulk containers without aggregation, to facilitate subsequent handling. Its production should minimise or eliminate concern about toxicological safety to plant personnel and, ultimately, the patient, resulting from the use of solvents which may be present, in small amounts, in the crystal lattice of the product. There should be mimimal by-product formation, e.g. as the result of local excesses of methanesulfonic acid, especially in the presence of water which may be introduced with the crystallisation solvents or in the crystal lattice of the adinazolam starting material.

A novel product of the invention is essentially non-hygroscopic adinazolam methanesulfonate. The product may be prepared in a non-hygroscopic, crystal form, essentially free of ring-opened by-product and undesirable solvent residues.

A process according to the invention, for preparing crystalline, essentially non-hygroscopic adinazolam methanesulfonate, comprises mixing adinazolam free base, methanesulfonic acid, a $C_{1-2}$ alkanol, i.e. methanol or ethanol and a $C_{4-6}$ alkyl acetate, e.g. butyl, pentyl or hexyl acetate, in which the molar ratio of methanesulfonic acid to adinazolam is no greater than 1;

heating the mixture to a temperature sufficient to dissolve some at least of the adinazolam free base and for a time sufficient to remove substantially all the alkanol and any water; and

cooling the resultant mixture and separating the precipitated crystalline adinazolam methanesulfonate.

Preferably, the mixture to be heated is formed by adding methanesulfonic acid to the adinazolam free base, alkanol and acetate.

The adinazolam methanesulfonate salt of this invention can be prepared from adinazolam free base or from various hydrated adinazolam acid salt forms, after freeing the adinazolam from any non-methanesulfonate salt forms.

The preferred, direct method for preparing essentially non-hygroscopic adinazolam methanesulfonate comprises mixing 1 to 15% by weight adinazolam free base into a liquid mixture of butyl acetate or pentyl acetate or hexyl acetate and $C_{1-2}$ alkanol. Preferably, the acetate:alkanol ratio is 99:1 to 85:15 v/v. This mixture is heated, is necessary, to effect solution of the adinazolam. Methanesulfonic acid diluted with a $C_1$ to $C_2$-alkanol is added in a slow controlled manner to minimise localised concentrations of methanesulfonic acid, to an amount just short of stoichiometric equivalency of the methanesulfonic acid relative to the adinazolam concentration in the mixture, and heating the mixture at a temperature sufficient to distill off the methanol or ethanol and essentially all of any water present in the mixture. Heating of these mixtures at a temperature of 110 to 120°C for $1\frac{1}{2}$ to several hours at atmospheric pressure is sufficient to remove the methanol or ethanol and essentially all of the water present in the mixture.

It has been found that it is necessary to add methanol or ethanol for the adinazolam free base or hydrated methanesulfonate salt thereof to the $C_4$ to $C_6$-alkyl acetate to increase the solubility of the adinazolam in the mixture. The amount of $C_1$ to $C_2$-alkanol added is not critical beyond providing the enhanced solubility property to the solvent mixture because the mixture is heated to distill or azeotrope off the $C_1$ to $C_2$-alkanol and any water in the mixture. Amounts of $C_1$ to $C_2$-alkanol ranging from about 10 to about 15 percent v/v, based upon the amount of $C_4$ to $C_6$-alkyl acetate, are generally sufficient. A solvent mixture containing about a 9:1 v/v $C_4$ to $C_6$-alkyl acetate or to $C_1$ to $C_2$-alkanol, is preferrred. Methanol is the preferred $C_1$ to $C_2$-alkanol. Also, maintaining the adinazolam free base concentration in the solvent mixture equal to or higher than the stoichiometric concentration of the methanesulfonic acid helps to ensure reproducibility of high quality, non-hygroscopic adinazolam methanesulfonate salt product yields. Such processing also minimizes formation of the yield lowering ring opened by-products in the mixture, probably due to the combined presence of excess methanesulfonic acid and water in the mixture, which conditions are sought to be avoided herein. The concentration of adinazolam free base or its equivalent hydrated adinazolam methanesulfonate starting material can be mixed with one or the other of the solvent mixture components, and then the other solvent component can be added. The concentration of the adinazolam in the solvent mixture can vary from dilute (concentrations of adinazolam down below or near 1 percent w/v adinazolam in solvent mixture volume) to more concentrated (concentration of adinazolam up near 15 percent w/v, on the same basis as above) for pilot or plant scale operation of the process.

Experiments have been conducted employing fast, one-shot or slow, dropwise, controlled addition of methanesulfonic acid to 1 percent and 10 percent, respectively, of adinazolam free base in a 9:1 v/v butyl acetate/methanol solvent mixture, at 23°C. until the adinazolam/methanesulfonic acid stoichiometric ratio in the mixture reached the indicated ratios, below.

2

## TABLE

| Run | Adinazolam/ 1% adinazolam/ volume (a) | Adinazolam: $H_3CSO_3H$ Ratio | Ring Addition Rate (e) | Ring Opened Impurity (b) |
|---|---|---|---|---|
| A. | | | | |
| I. | | 1.05 | Fast | LT[d] 0.2% |
| II. | | 0.83 | Fast | ~[d] 8% |
| III. | | 1.00 | Fast | LT 0.2% |
| IV. | | 0.91 | Slow | ~ 6% |
| B. | 10% adinazolam/ volume | | | |
| I. | | 0.83 | Fast | 3.8% |
| II. | | 1.04 | Fast | LT 0.2% |
| III. | | 0.92 | Slow | 4.1% |
| IV. (c) | | 1.01 | Fast | 0.5% |
| V. | | 1.01 | Fast | LT 0.1% |

Footnotes:

a - All runs conducted in solvent mixture of butyl acetate/-methanol

b - Area percent, based upon adinazolam content

c - This adinazolam starting material contained 3 percent w/v of N-[[4-(2-benzoyl-4-chlorophenyl)-5-[(dimethylamino)methyl]-4H-1,2,4-triazol-3-yl]methyl]phthalimide

d - LT = less than; ~ = about.

e - Fast = methane sulfonic acid added rapidly in a single portion

Slow = methane sulfonic acid added in a controlled dropwise fashion

The studies at the lower concentration levels allowed more uniform sampling of the reaction slurries; the data for the higher concentration studies are more in line with expected plant conditions. Data from both low and higher concentration studies are reasonably consistent; slight variations in the semiquantitative results can be attributed to non-uniform sampling of the mixtures.

From these data, it is significant that in cases where adinazolam free base was present even in slight excess, relative to the molar content of methanesulfonic acid, the presence of ring-opened impurity in the product was always less than 0.2 percent. A leveling of the amount of ring-opened impurity is seen over time since any localized regions of high methanesulfonic acid acidity are neutralized as the hot slurry thins. The data show that in runs where the methanesulfonic acid is present in excess, relative to the adinazolam free base content, without exception, unacceptably high levels of the ring-opened impurity were found in the isolated adinazolam methanesulfonate product. To avoid problems associated with the presence of ring-opened impurity in isolating the crystalline non-hygroscopic adinazolam methanesulfonate product, therefore, it is desirable to maintain the stoichiometric ratio of methanesulfonic acid to adinazolam in these dehydrating solvent systems to less than 1, that is, the amount of methanesulfonic acid used is deliberately kept slightly lower or no higher than its molar equivalent of adinazolam free base.

As used herein, non-hygroscopic adinazolam methanesulfonate salt is salt which does not have water of hydration in its crystal structure above about 0.15 percent, by weight, or which does not take up water from the atmosphere, or which does not form hard clumps of crystal aggregates of adinazolam methanesulfonate salt upon standing in storage containers awaiting further processing.

Another, but less preferred emthod or process for preparing non-hygroscopic adinazolam methanesulfonate salt is to dissolve adinazolam free base in methanol, with the aid of heat, if necessary, for the concentration of adinazolam in methanol which is desired, cool the solution to about room temperature, add the methanesulfonic acid in methanol to form the hydrated adinazolam methanesulfonate salt, which precipitates when the mixture is cooled to about 0°C. The precipitated hydrated adinazolam methanesulfonate salt can be separated from the lqiuid phase and mixed with a high boiling liquid (i.e., a b.p. over about 110°C) such as xylene, toluene, cyclohexanone, or Skellysolve ®V (a brand of petroleum sovlent, which consists essentially of mixed $C_8$ and $C_9$ aliphatic hydrocarbons, boiling at about 240°—290°C). Xylene is representative of this group. The adinazolam methanesulfonate/high boiling solvent mixture is set for distillation, and the mixture is heated until water or a water/solvent azeotrope ceases to be collected in condensing apparatus connected thereto. Then the mixture is cooled to room temperature or below to precipitate and crystallize the non-hygroscopic adinazolam methanesulfonate salt, which can then be separated and dried by conventional means to a desired degree of dryness.

This latter method is not preferred because approximately 0.1 to about 0.2 percent by weight of xylene or other solvent can be detected in this type of non-hygroscopic adinazolam methanesulfonate salt crystal product. Recent investigations by persons concerned with toxicological and occupational safety have recommended xylene not be used, but that butyl acetate, or a $C_5$ or $C_6$-alkyl acetate would be preferred over the use of the other above named solvents for this recrystallization process. Residual solvent levels of either butyl acetate, or a $C_5$ or $C_6$-alkyl acetate, not exceeding 0.1 percent by weight, thereof, based upon the weight of the adinazolam in the finished non-hydroscopic adinazolam methanesulfonate crystalline product are not considered to represent a significant toxicological hazard at this time. However, efforts should be made to minimize, if not eliminate, the presence of any of these residual solvents.

The following examples illustrate the invention.

## Example 1

In a 10 ml. round bottomed flask fitted with a magnetic stir bar and a condenser there was placed 150 mg of hydrated adinazolam methanesulfonate salt and 5 ml of reagent grade xylene. The stirring mixture was heated in an oil bath over 1.33 hr to reflux, and maintained at reflux temperature for about 1 hour to remove water from the mixture. The oil bath temperature varied from about 120°C to 165°C. The resulting mixture was then cooled to 20°C, the adinazolam methanesulfonate solids were removed by filtration, washed with 1 ml of reagent grade xylene, dried in a vacuum oven at about 50°C for about 16 hours to constant weight, about 140 mg. The filtration was excellent. The yield of non-hygroscopic adinazolam methanesulfonate was thus about 93 percent; m.p. 242°—245°C, a brown melt.

## Example 2

This example exemplifies the procedure of Example 1, using xylene, on a larger scale.

To a 1-liter 3-necked round-bottomed flask there was added 48.98 g of hydrated adinazolam methanesulfonate and 750 ml of reagent grade xylene. The slurry was heated and stirred at reflux, about 136°C, for 2 hours. A Dean-Stark trap apparatus was used to azeotropically remove any water present. Only a few drops of water was collected from this batch of adinazolam methanesulfonate starting material. However, the distillate was turbid when cooled. The resulting slurry was cooled over 1.33 hour to about +15°C and the adinazolam methanesulfonate salt was collected by filtration. The filtration was excellent. The filtered salt was washed with 100 ml. of room temperature xylene. The washed salt was then air dried at room temperature for ten minutes and then dried in a vacuum oven at 102°C for 136 hours to give 44.55 g, 95 percent recovery of adinazolam methanesulfonate non-hygroscopic salt, m.p. 244°—245°C.

## Example 3

To a flask apparatus of the type described in Example 1 there was added 150 mg of hydrated adinazolam methanesulfonate and 5 ml of reagent grade butyl acetate. The mixture was heated on an oil bath to reflux for about 1 hour and then cooled to 20°C. The solids were collected by filtration, wahed with 1 ml of butyl acetate, and dried in a vacuum oven at about 50°C for 16 hours to constant weight of 131 mg. The filtration was excellent. The yield was about 87 percent, but with cooling the yield should be about equivalent to that of xylene. The excellent filtration was about comparable to that of the adinazola-methanesulfonate salt from xylene. Butyl acetate or butanol can be used to replace the use of xylene in such crystallization to obtain non-hygroscopic adinazolam methanesulfonate.

## Example 4

To a 5-liter 3-neck round-bottom flask equipped with a distillation head, a nitrogen gas inlet, an addition funnel, an overhead stirrer and a hot oil bath heating means there was added 300 g. of adinazolam free base, 3.6 liters of n-butyl acetate and 300 ml of methanol. The resulting mixture was warmed to 30°C to dissolve all solids. Then, while stirring rapidly, 77.7 g of methanesulfonic acid was added slowly via the dropping funnel. The resulting mixture was stirred, and the internal temperature of the flask contents was gradually raised to 110°C while monitoring the amount of methanol distillate coming from the reaction vessel. After 0.5 hours at an internal temperature of 110°—115°C. (with no further observable distillate coming over), a sample of the resulting reaction mixture is removed, the solid salt thereof is isolated, and its melting point is checked. Any melting activity in the temperature range of 180°—195°C indicates incomplete conversion of adinazolam to its desired non-hygroscopic adinazolam methanesulfonate salt form. When the melting point of the solid salt sample is in the range of 242°—246°C, the salt forming reaction and crystal form interchange is complete.

When the reaction is complete, the resulting mixture is cooled to ambient temperature, filtered to separate the solid adinazolam methanesulfonate non-hygroscopic salt product, washed with butyl acetate and dried in a vacuum oven at 50°C overnight. The resulting non-hygroscopic adinazolam methanesulfonate salt is profile quality, at least 99.6 percent pure by high pressure liquid chromatography analyses means. The yield is about 361 g, 98 percent, based on methanesulfonic acid.

## Example 5 LARGE SCALE

To a large reaction vessel, there is charged 4.44 kg. (12.61 moles) of adinazolam free base. The atmosphere of the vessel is rendered inert by removing air therefrom with a nitrogen gas flow and then 60 kg of methanol is pulled into the inerted reaction vessel. The resulting mixture is heated to 40°C whereupon a solution develops. The solution is filtered at 40°C through a sterile filter pad followed by a 2 gallon methanol rinse. The filtered solution is transferred back into the clean reaction vessel, cooled to 25°C and then treated over 5 minutes with a solution of 1,263 g (13.14 mole) of methanesulfonic acid in 7.3 liters of methanol. At the end of the addition of the methanesulfonic acid solution, the mixture is cooled to 0°C is begun. After about 10 minutes of cooling a white precipitate will be observed which thickens somewhat and then thins out. The reactor vessel contents are filtered at 0°C to a damp filter cake. The filter cake (hygroscopic form of adinazolam methanesulfonate salt) is transferred back to the reaction vessel and mixed with 25—30 gallons of xylene. The resulting mixture in the vessel is set for distillation, and the mixture is heated and distilled (with the use free of free steam in the vessel jacket, and then with pressure steam in the vessel jacket) until the reaction vessel pot temperature is 135°C. Distillation starts at about 70°C with a water/xylene azeotrope distillate. At 135°C, the reaction vessel and its contents are switched to a reflux mode and refluxed for 0.5 hour at 135°C. The contents of the reaction vessel are then cooled slowly to room temperature. The contents of the vessel are then filtered and washed with 3 gallons of Skellysolve®B brand of mixed hexanes. The filtered solids, non-hygroscopic adinazolam methanesulfonate salt are dried *in vacuo* at 70°C for 2 days. The yield of non-hygroscopic adinazolam methanesulfonate salt from this procedure is about 4.44 to 78.6 percent. The characteristic non-hygroscopic form of adinazolam methanesulfonate is shown by an infrared (IR) spectrum of a sample of this salt form product.

## Example 6 LARGE SCALE, PREFERRED PROCESS

To a large, pilot plant scale, stainless steel reactor vessel, from which air is removed with nitrogen gas, there is added 9.2 kg. of adinazolam free base via the reactor manhole, while using a ventilation duct. The chemical operator should use gloves, a respirator and face shield protection equipment. The reactor manhole cover is closed and the reactor vessel is evacuated of air by flushing with nitrogen. Then 96.8 kg of n-butyl acetate is pulled into the reactor vessel by vacuum from a grounded drum. Then 7.3 kg of absolute methanol is pulled into the reactor vessel from a grounded drum. The reactor vessel contents are stirred and heated to 35°C to dissolve the adinazolam solids in the mixture. While continuing to heat the mixture 2.382 kg of methanesulfonic acid is pulled into the reaction vessel with vacuum from a glass bottle connected with polyethylene tubing. The vacuum is broken with nitrogen gas.

The resulting mixture is stirred rapidly (the mixture becomes thick), and is heated slowly to 110°C, while methanol and any water in the mixture is distilled off.

After 2 hours of heating the stirred mixture at 110°C a sample of the reaction mixture is taken and checked by melting point for completion of the reaction. When the melting point of the adinazolam methanesulfonate salt in the sample is 242°—246°C, the reaction is considered complete. The solids should be free-flowing and settle quickly when the reaction is complete and stirring is discontinued.

The resulting reaction mixture is cooled to room temperature and the solids are filtered via a 20-inch stainless steel, grounded filter. The filtered solids are rinsed with butyl acetate. The resulting crystalline adinazolam methanesulfonate salt is dried in a vacuum oven at 50°C for three days. The yield of non-hygroscopic adinazolam methanesulfonate is 11.0 kg (94 percent yield).

**Claims**

1. Crystalline adinazolam methanesulfonate which is essentially non-hygroscopic.

2. A process for preparing crystalline, essentially non-hygroscopic adinazolam methanesulfonate, which comprises:

(i) forming a mixture of adinazolam free base, methanesulfonic acid, a $C_{1-2}$ alkanol and a $C_{4-6}$ alkyl acetate, in which the molar ratio of methanesulfonic acid to adinazolam is no greater than 1;

(ii) heating the mixture to a temperature sufficient to dissolve some at least of the adinazolam free base and for a time sufficient to remove substantially all the alkanol and any water; and

(iii) cooling the mixture and separating the precipitated crystalline adinazolam methanesulfonate.

3. A process according to claim 2, in which the mixture is formed by adding methanesulfonic acid to the adinazolam free base, alkanol and acetate.

**Patentansprüche**

1. Kristallines Adinazolammethanesulfonat, das im wesentlichen nicht-hygroskopisch ist.

2. Ein Verfahren zur Herstellung von kristillinem, im wesentlichen nicht-hygroskopischem Adinazolammethansulfonat, das die folgenden Stufen umfaßt:

(i) Bildung eines Gemisches von adinazolamfreier Base, Methansulfonsäure, einem $C_{1-2}$ Alkanol und einem $C_{4-6}$ Alkylacetat, bei dem das Molekularverhältnis der Methansulfonsäure zum Adinazolam nicht höher ist als 1;

(ii) Erhitzung des Gemisches auf eine Temperatur, die genügend hoch ist, um mindestens die adinazolamfreie Base aufzulösen, und eine Zeitlang genügend hoch ist, um im wesentlichen das ganze Alkohol und etwaiges Wasser zu beseitigen; und

(iii) Kühlung des Gemisches und Trennung des gefällten kristallinen Adinazolammethansulfonats.

3. Ein Verfahren im Einklang mit Anspruch 2, bei dem das Gemisch durch Zusatz von Methansulfonsäure zu der adinazolamfreien Base, dem Alkanol und dem Acetat gebildet wird.

**Revendications**

1. Méthanesulfonate d'adinazolam cristallin, qui est pratiquement non hygroscopique.

2. Procédé de préparation de méthanesulfonate d'adinazolam cristallin, pratiquement non hygroscopique, qui consiste:

(i) à préparer un mélange d'adinazolam sous forme de base libre, d'acide méthanesulfonique, d'un alcanol en $C_1$ ou $C_2$ et d'un acétate d'alkyle en $C_4$ à $C_6$, dans lequel le rapport molaire de l'acide méthanesulfonique à l'adinazolam est non supérieur à 1;

(ii) à chauffer le mélange à une température suffisante pour dissoudre au moins une certaine quantité de l'adinazolam sous forme de base libre et pendant un temps suffisant pour éliminer pratiquement la totalité de l'alcanol et toute quantité d'eau; et

(iii) à refroidir le mélange et à séparer le méthanesulfonate d'adinazolam cristallin précipité.

3. Procédé suivant la revendication 2, dans lequel le mélange est préparé par addition d'acide méthanesulfonique à l'adinazolam sous forme de base libre, l'alcanol et l'acétate.